# EUROPEAN PATENT APPLICATION

(11) **EP 2 351 546 A1**
(43) Date of publication of application: **03.08.2011**
(21) Application number: 09827490.5
(22) Date of filing: 09.11.2009
(51) Int. Cl.: A61F 13/15, A61F 13/49, A61F 13/53

(54) **DEVICE AND METHOD FOR MANUFACTURING ABSORBING BODY**

(30) Priority: 20.11.2008 JP 2008296753
(71) Applicant: Uni-Charm Corporation, Ehime 799-0111 (JP)
(72) Inventor: YANO, Takanori, Kanonji-shi Kagawa 769-1602 (JP)
(74) Representative: Peter, Julian
(86) International application number: PCT/JP2009/069035
(87) International publication number: WO 2010/058709

(57) **Abstract**

An apparatus for manufacturing 10 an absorbent body 1 including a forming member having a predetermined face 20a with a recessed form die 21 formed thereon, moving the form die 21 in one direction along a moving path intersecting a width direction of the predetermined face 10a; a supply duct 31 disposed in a predetermined position in the moving path, supplying a gas 3 including fluid absorbent fibers 2 from a supply opening section 31a toward the predetermined face 20a; a negative pressure room partition member 25 provided on a side of the predetermined face 20a, with the supply opening section 31a provided on an opposite side of the predetermined face 20a, the negative pressure room partition member 25 partitioning a negative pressure room S in cooperation with the predetermined face 20a; and an exhaust duct 41 exhausting a gas 3 inside the negative pressure room S so as to create a negative pressure inside the negative pressure room S, wherein in a case of the form die 21 passing a position of the supply opening section 31a, by a gas 3 from the supply duct 31 being sucked through air intake holes in a bottom section 21a of the form die 21 into the negative pressure room S, the fluid absorbent fibers 2 in the gas 3 are stacked in the form die 21 to form an absorbent body 1. The negative pressure room partition member 25 includes a pair of wall sections 25, 25 disposed by sandwiching therebetween the moving path in the width direction, and the negative pressure room S being partitioned between the pair of wall sections 25, 25. The exhaust duct 41 is connected to one wall section 25 of the pair of wall sections 25, 25 with an exhaust opening section 42 of the exhaust duct 41 provided by projecting inward beyond the wall section 25 into the negative pressure room S.

## Description

### Technical Field

The present invention relates to an apparatus and a method for manufacturing an absorbent body of an absorbent article such as a disposable diaper.

### Background Art

As examples of an absorbent article that absorbs fluid, disposable diapers, sanitary napkins, and the like are used. These absorbent articles include an absorbent body 1 that is produced by forming pulp fibers into a predetermined shape.

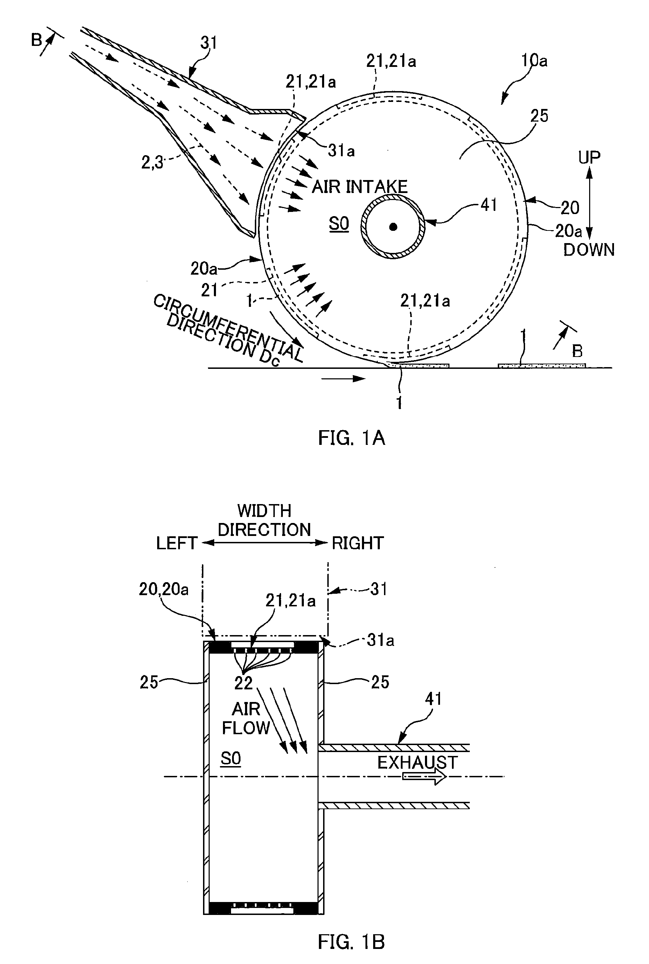

The absorbent body 1 is formed by a fiber stacking apparatus 10a in a production line. FIG. 1A is a side view of the fiber stacking apparatus 10a partially shown in a cross-section. FIG. 1B is a cross-sectional view taken from line B-B of FIG. 1A. The fiber stacking apparatus 10a has a rotating drum 20. The rotating drum 20 includes as a main body a cylindrical member that rotates in a circumferential direction Dc. Openings at both ends of the rotating drum 20 in a width direction (a direction perpendicular to the plane of the paper in FIG. 1A) are covered with a pair of circular wall sections 25, 25 and thus sealed, and thereby a negative pressure room SO is partitioned on an inner circumferential side of the rotating drum 20. Recessed form dies 21 are formed on an outer circumferential surface 20a of the rotating drum 20, and a plurality of air intake holes 22 that communicatively connect a bottom section 21a of the form die 21 to the negative pressure room SO are formed in the bottom section 21a. And a supply duct 31 that discharges an air mixture 3 with the pulp fibers 2 mixed therein is disposed so as to face the outer circumferential surface 20a of the rotating drum 20.

Thus, in the case of the form die 21 passing the position of the supply duct 31 as the rotating drum 20 rotates, the air mixture 3 discharged from the supply duct 31 is sucked through the bottom section 21a of the form die 21. Thereby, the pulp fibers 2 in the air mixture 3 are stacked inside the form die 21 and thus formed into the absorbent body 1.

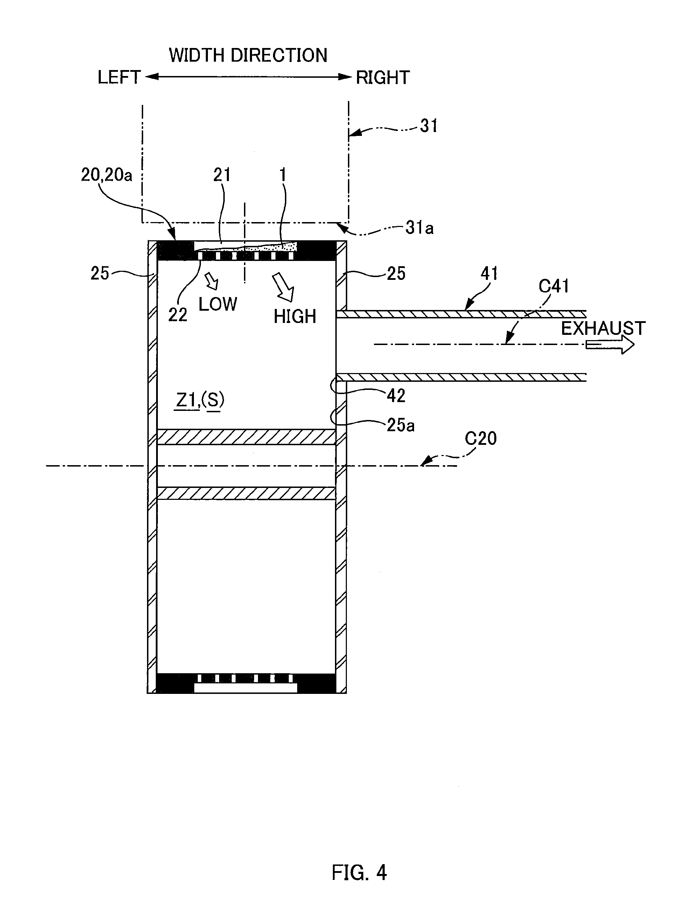

However, a negative pressure state in the negative pressure room SO on the inner circumferential side of the rotating drum 20 is, as shown in FIG. 1A, created by an exhaust duct 41 connected to the circular wall section 25 exhausting an air inside the negative pressure room SO. However, usually the exhaust duct 41 is connected to only one wall section 25 of the pair of circular wall sections 25, 25 provided in the width direction as shown in FIG. 1B, and therefore a suction force in the form die 21 is biased in the width direction of the rotating drum 20. And as a result, stack distribution of the absorbent body 1 in the form die 21 is biased in the width direction. For example, in an example of FIG. 1B, the absorbent body 1 in the right side part of the form die 21 to which the exhaust duct 41 is connected would be stacked high, while the left side part that is on the opposite side in FIG. 1B would be stacked low (refer to FIG. 4).

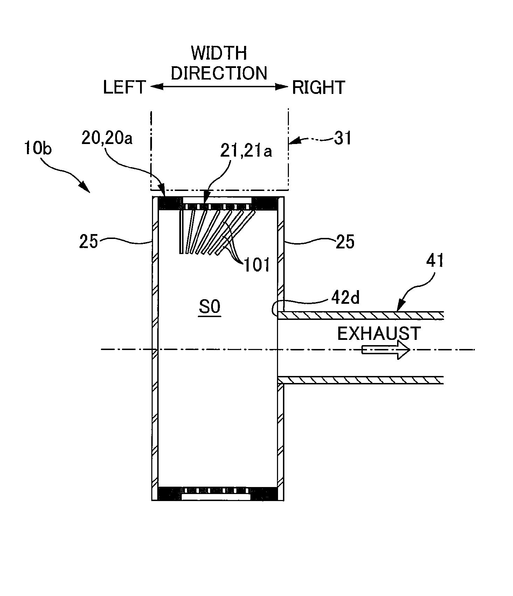

Regarding this point, in PTL 1, to prevent such non-uniform stack distribution, a plurality of flow adjusting plates 101 are provided between an exhaust opening section 42d of the exhaust duct 41 connected to the circular wall section 25 and the rotating drum 20 as shown in FIG. 2. And technology to prevent the non-uniform stack distribution by making path lengths from each of all positions in the form die 21 in the width direction to the exhaust opening section 42d equal is disclosed.

### Citation List

### Patent Literature

PTL 1: JP-A-2007-202640

### Summary of Invention

### Technical Problem

However, the configuration of an apparatus gets complicated by providing the plurality of flow adjusting plates 101.

The present invention was made in view of the foregoing conventional problem, and it is an advantage thereof to provide an apparatus and a method for manufacturing an absorbent body that can make the stack distribution of the absorbent body uniform with a quite simple configuration.

### Solution to Problem

A main aspect of the invention for achieving the foregoing object is
an apparatus for manufacturing an absorbent body, including:
a forming member having a predetermined face with a recessed form die formed thereon, moving the form die in one direction along a moving path intersecting a width direction of the predetermined face;
a supply duct disposed in a predetermined position in the moving path, supplying a gas including fluid absorbent fibers from a supply opening section toward the predetermined face;
a negative pressure room partition member provided on a side of the predetermined face, with the supply opening section provided on an opposite side of the predetermined face, the negative pressure room partition member partitioning a negative pressure room in cooperation with the predetermined face; and
an exhaust duct exhausting a gas inside the negative pressure room so as to create a negative pressure inside the negative pressure room,
wherein in a case of the form die passing a position of the supply opening section, by a gas from the supply duct being sucked through air intake holes in a bottom section of the form die into the negative pressure room, the fluid absorbent fibers in the gas are stacked in the form die to form an absorbent body,
the apparatus further having
   the negative pressure room partition member including a pair of wall sections disposed by sandwiching therebetween the moving path in the width direction, the negative pressure room being partitioned between the pair of wall sections, and
   the exhaust duct connected to one wall section of the pair of wall sections, with an exhaust opening section of the exhaust duct provided by projecting inward beyond the wall section into the negative pressure room.

And another main aspect of the invention for achieving the foregoing object is
a method for manufacturing an absorbent body, including:
(A) preparing an apparatus for manufacturing an absorbent body, the apparatus for manufacturing the absorbent body using
   a forming member having a predetermined face with a recessed form die formed thereon, moving the form die in one direction along a moving path intersecting a width direction of the predetermined face;
   a supply duct disposed in a predetermined position in the moving path, supplying a gas including fluid absorbent fibers from a supply opening section toward the predetermined face;
   a negative pressure room partition member provided on a side of the predetermined face, with the supply opening section provided on an opposite side of the predetermined face, the negative pressure room partition member partitioning a negative pressure room in cooperation with the predetermined face; and
   an exhaust duct exhausting a gas inside the negative pressure room so as to create a negative pressure inside the negative pressure room,
   wherein in a case of the form die passing a position of the supply opening section, by a gas from the supply duct being sucked through air intake holes in a bottom section of the form die into the negative pressure room, the fluid absorbent fibers in the gas are stacked in the form die to form the absorbent body,
   the apparatus further having
   the negative pressure room partition member including a pair of wall sections disposed by sandwiching therebetween the moving path in the width direction, the negative pressure room being partitioned between the pair of wall sections, and
   the exhaust duct connected to one wall section of the pair of wall sections, with an exhaust opening section of the exhaust duct provided by projecting inward beyond the wall section into the negative pressure room; and
(B) manufacturing the absorbent body using the apparatus for manufacturing the absorbent body.

Other features of the invention will become clear by the description of the present specification and the accompanying drawings.

### Advantageous Effects of Invention

According to an aspect of the invention, an absorbent body can make stack distribution of the absorbent body uniform with a quite simple configuration.

### Brief Description of Drawings

FIG. 1A is a side view of a fiber stacking apparatus 10a partially shown in a cross-section.
FIG. 1B is a cross-sectional view taken from line B-B of FIG. 1A.
FIG. 2 is a cross-sectional view of a conventional fiber stacking apparatus 10b by which stack distribution can be made uniform.
FIG. 3A is a center vertical cross-sectional view of a fiber stacking apparatus 10 according to the first embodiment.
FIG. 3B is a cross-sectional view taken from line B-B of FIG. 3A.
FIG. 4 is an explanatory diagram showing problems of the fiber stacking apparatus.
FIGS. 5A and 5B are cross-sectional views of the fiber stacking apparatus 10 according to modified examples of the first embodiment.
FIG. 6A is a center vertical cross-sectional view of the fiber stacking apparatus 10 according to the second embodiment.
FIG. 6B is a cross-sectional view taken from line B-B of FIG. 6A.
FIG. 7 is a perspective view of an exhaust duct 41 according to the second embodiment.
FIG. 8 is a perspective view of another example of the exhaust duct 41 according to the second embodiment.
FIG. 9A is a center vertical cross-sectional view of another example of the fiber stacking apparatus 10 according to the second embodiment.
FIG. 9B is a cross-sectional view taken from line B-B of FIG. 9A.
FIG. 10A is a center vertical cross-sectional view of another example of the fiber stacking apparatus 10 according to the second embodiment.
FIG. 10B is a cross-sectional view taken from line B-B of FIG. 10A.
FIG. 11 is an explanatory diagram showing a reason why a flowing speed of an air flow decreases at a pipe end 41a side of an exhaust opening section 42 of the exhaust duct 41.
FIGS. 12A and 12B are explanatory diagrams of permissible area of a disposing position of the exhaust opening section 42 corresponding to a width direction of a rotating drum 20.
FIG. 13 is an explanatory diagram showing a variation in the number of the exhaust duct 41 and the like.
FIG. 14A is a center vertical cross-sectional view of a fiber stacking apparatus 10 according to the other embodiment.
FIG. 14B is a cross-sectional view taken from line B-B of FIG. 14A.
FIG. 15 is a cross-sectional view of the fiber stacking apparatus 10 according to another embodiment.

### Description of Embodiments

At least the following matters will be made clear by the description in the present specification and the accompanying drawings.

An apparatus for manufacturing an absorbent body, including:
a forming member having a predetermined face with a recessed form die formed thereon, moving the form die in one direction along a moving path intersecting a width direction of the predetermined face;
a supply duct disposed in a predetermined position in the moving path, supplying a gas including fluid absorbent fibers from a supply opening section toward the predetermined face;
a negative pressure room partition member provided on a side of the predetermined face, with the supply opening section provided on an opposite side of the predetermined face, the negative pressure room partition member partitioning a negative pressure room in cooperation with the predetermined face; and
an exhaust duct exhausting a gas inside the negative pressure room so as to create a negative pressure inside the negative pressure room,
wherein in a case of the form die passing a position of the supply opening section, by a gas from the supply duct being sucked through air intake holes in a bottom section of the form die into the negative pressure room, the fluid absorbent fibers in the gas are stacked in the form die to form an absorbent body,
the apparatus further having
the negative pressure room partition member including a pair of wall sections disposed by sandwiching therebetween the moving path in the width direction, the negative pressure room being partitioned between the pair of wall sections, and
the exhaust duct connected to one wall section of the pair of wall sections, with an exhaust opening section of the exhaust duct provided by projecting inward beyond the wall section into the negative pressure room.

According to such an apparatus for manufacturing an absorbent body, the exhaust opening section of the exhaust duct is provided by projecting inward to the negative pressure room beyond the wall section. Thus sucking-pressure distribution of the gas can be made uniform in the width direction than in the case where the exhaust opening section is positioned in the wall section. As a result, the stack distribution of the absorbent body can be made uniform in the width direction.

In the apparatus for manufacturing an absorbent body, it is preferable that the exhaust duct is a tubular member and the exhaust opening section is formed in a circumferential wall section of the exhaust duct.

According to such an apparatus for manufacturing an absorbent body, the exhaust opening section is formed in a circumferential wall section of the exhaust duct and thus a distance between the bottom section of the form die and the exhaust opening section can be made substantially equal over the width direction, and as a result, the stack distribution of the absorbent body can be made uniform in the width direction.

Also, in the case of the form die passing a position close to the exhaust opening section, the exhaust opening section can suck gas along the direction substantially normal to the bottom section of the form die and the stack distribution of the absorbent body can be made uniform also in this way.

In the apparatus for manufacturing an absorbent body, it is preferable that a pipe axis direction of a portion of the exhaust duct positioned in the negative pressure room is parallel to the predetermined face and the bottom section of the form die.

According to such an apparatus for manufacturing an absorbent body, a distance between the bottom section of the form die and the exhaust opening section can be made equal over the width direction, and as a result, the stack distribution of the absorbent body can be made uniform in the width direction.

Also in the case of the form die passing a position close to the exhaust opening section, the exhaust opening section can suck gas along the direction normal to the bottom section of the form die. And the stack distribution of the absorbent body can be made uniform also in this way.

In the apparatus for manufacturing an absorbent body, it is preferable that a portion at which the exhaust opening section is formed in a circumferential direction of the exhaust duct is a portion on an opposite side of a portion opposing the supply opening section.

According to such an apparatus for manufacturing an absorbent body, a gas that has passed the supply opening section and the air intake holes at the bottom section of the form die is sucked to encircle the exhaust duct and thereafter, through the exhaust opening section on the opposite side of the bottom section of the form die. Thus, even in the case where the sucking pressure at the exhaust opening section varies to some degree in the width direction, effects by such pressure variation become smaller at the position of the bottom section of the form die, which makes the sucking-pressure distribution in the form die in the width direction is made further uniform.

In the apparatus for manufacturing an absorbent body, it is preferable that in a case where the exhaust opening section is a first exhaust opening section, a second exhaust opening section is formed in a portion opposing the supply opening section in the circumferential wall section of the exhaust duct.

According to such an apparatus for manufacturing an absorbent body, increase of pressure loss incidents to the first exhaust opening section being provided in a portion farthest from the supply opening section can be reduced by the second exhaust opening section.

In the apparatus for manufacturing an absorbent body, it is preferable that an opening area of the second exhaust opening section is smaller than an opening area of the first exhaust opening section.

According to such an apparatus for manufacturing an absorbent body, the opening area of the second exhaust opening section is smaller than the opening area of the first exhaust opening section so the second exhaust opening section is used subsidiarily. Thereby, the pressure loss that may occur can be compensated while efficiently maintaining the effect of uniformizing the sucking-pressure distribution in the form die in the width direction caused by gas sucking from the first exhaust opening section.

In the apparatus for manufacturing an absorbent body, it is preferable that an end of the exhaust duct in the pipe axis direction is blocked by a blocking member, and
an existence of the circumferential wall section of the exhaust duct between the end and the exhaust opening section causes a gas stagnation at an end portion of the exhaust duct in the pipe axis direction.

According to such an apparatus for manufacturing an absorbent body, decrease of the flow rate of the gas can be reduced by the gas stagnation at the end, and as a result, sucking-pressure distribution in the exhaust opening section in the width direction can be made uniform.

In the apparatus for manufacturing an absorbent body, it is preferable that the bottom section of the form die and the exhaust opening section are disposed to at least overlap in the width direction.

According to such an apparatus for manufacturing an absorbent body, the exhaust opening section can suck gas along the direction substantially normal to the bottom section of the form die. Thus the stack distribution of the absorbent body can be made uniform.

In the apparatus for manufacturing an absorbent body, it is preferable that a center position of the exhaust opening section in the width direction is positioned in the bottom section of the form die.

According to such an apparatus for manufacturing an absorbent body, the exhaust opening section can suck gas along the direction substantially normal to the bottom section of the form die efficiently. Thus the stack distribution of the absorbent body can be made uniform.

In the apparatus for manufacturing an absorbent body, it is preferable that the center position of the exhaust opening section in the width direction aligns with a center position of the bottom section of the form die.

According to such an apparatus for manufacturing an absorbent body, the exhaust opening section can suck gas along the direction substantially normal to the bottom section of the form die efficiently. Thus the stack distribution of the absorbent body can be made uniform.

In the apparatus for manufacturing an absorbent body, it is preferable that the forming member is a cylindrical member that continuously rotates in one circumferential direction,
the recessed form die is formed on an outer circumferential surface of the cylindrical member as the predetermined face, moved by rotation of the cylindrical member in the circumferential direction in a path along the circumferential direction as the moving path,
a pair of circular wall sections are included as the pair of wall sections of the negative pressure room partition member, the pair of circular wall sections covering openings at both ends of the cylindrical member in the width direction to partition the negative pressure room on an inner circumferential side of the cylindrical member, and
the exhaust duct is connected to one wall section of the pair of circular wall sections.

According to such an apparatus for manufacturing an absorbent body, effects of the invention of the present application can be achieved in an effective manner.

Also a method for manufacturing an absorbent body, including:
(A) preparing an apparatus for manufacturing an absorbent body, the apparatus for manufacturing the absorbent body using a forming member having a predetermined face with a recessed form die formed thereon, moving the form die in one direction along a moving path intersecting a width direction of the predetermined face;
   a supply duct disposed in a predetermined position in the moving path, supplying a gas including fluid absorbent fibers from a supply opening section toward the predetermined face;
   a negative pressure room partition member provided on a side of the predetermined face, with the supply opening section provided on an opposite side of the predetermined face, the negative pressure room partition member partitioning a negative pressure room in cooperation with the predetermined face; and
   an exhaust duct exhausting a gas inside the negative pressure room so as to create a negative pressure inside the negative pressure room,
   wherein in a case of the form die passing a position of the supply opening section, by a gas from the supply duct being sucked through air intake holes in a bottom section of the form die into the negative pressure room, the fluid absorbent fibers in the gas are stacked in the form die to form the absorbent body,
   the apparatus further having
   the negative pressure room partition member including a pair of wall sections disposed by sandwiching therebetween the moving path in the width direction, the negative pressure room being partitioned between the pair of wall sections, and
   the exhaust duct connected to one wall section of the pair of wall sections, with an exhaust opening section of the exhaust duct provided by projecting inward beyond the wall section into the negative pressure room; and
(B) manufacturing the absorbent body using the apparatus for manufacturing the absorbent body.

According to such a method for manufacturing an absorbent body, the exhaust opening section of the exhaust duct is provided by projecting inward to the negative pressure room beyond the wall section. Thus sucking-pressure distribution of the gas is made uniform in the width direction than in the case where the exhaust opening section is positioned in the wall section. As a result, the stack distribution of the absorbent body can be made uniform in the width direction.

### Embodiments

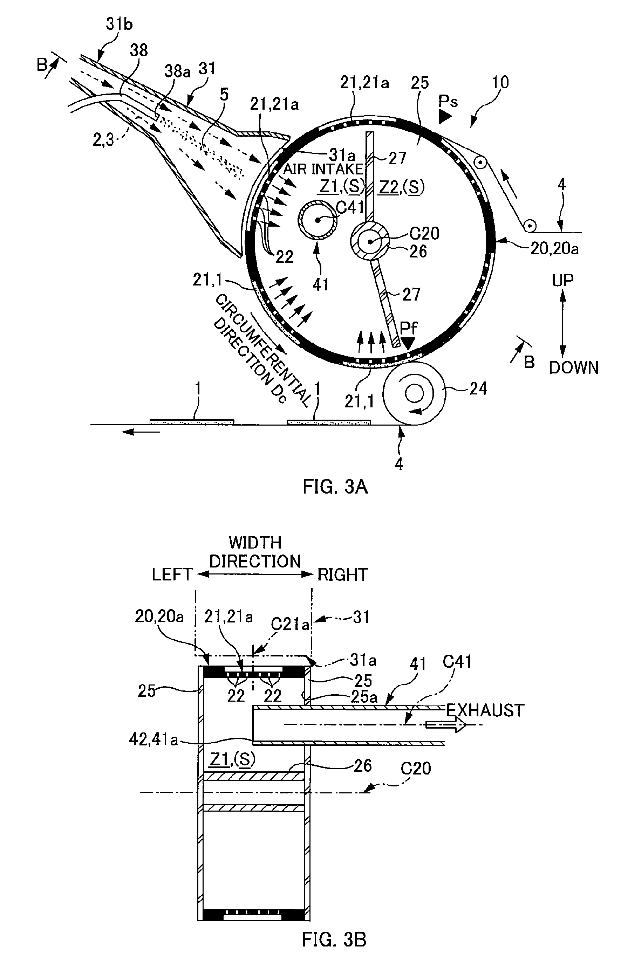

FIG. 3A is a center vertical cross-sectional view of an apparatus for manufacturing 10 of an absorbent body 1 according to the first embodiment. FIG. 3B is a cross-sectional view taken from line B-B of FIG. 3A.

The apparatus for manufacturing 10 of the absorbent body 1 is a so-called fiber stacking apparatus, which stacks pulp fibers 2 as fluid absorbent fibers to thereby form the absorbent body 1. As its main configuration, the apparatus for manufacturing 10 includes (1) a rotating drum 20 (corresponds to forming member) that continuously rotates in one direction of a circumferential direction Dc (for example, counterclockwise direction) about a horizontal axis C20, (2) a supply duct 31 that discharges and supplies an air mixture 3 (corresponds to a gas) including the pulp fibers 2 from a supply opening section 31a disposed at a predetermined position in the circumferential direction Dc of the rotating drum 20 toward the outer circumferential surface 20a of the rotating drum 20.

Note that, hereinafter, the circumferential direction Dc of the rotating drum 20 is simply referred to as a "circumferential direction" and a direction along the horizontal axis C20 of the rotating drum 20 (direction perpendicular to the plane of the paper in FIG.3A) is referred to as a "width direction" or a "right and left direction".

The rotating drum 20 includes a cylindrical member that rotates about the horizontal axis C20 as a main body. Openings in both ends of the rotating drum 20 in the width direction are covered with a pair of circular wall sections 25, 25 (correspond to negative pressure room partition member or wall section) and thus blocked. And inside the rotating drum 20, a cylindrical division wall 26 is provided concentrically with the rotating drum 20, and thereby a doughnut-shaped substantially closed space S is partitioned in the inner circumferential side of the rotating drum 20.

And recessed form dies 21 corresponding to a shape of the absorbent body 1 to be formed are intermittently provided on the outer circumferential surface 20a of the rotating drum 20 (corresponds to a predetermined face) that is parallel to the width direction at a predetermined pitch in the circumferential direction Dc. Each bottom section 21a of the form dies 21 is formed parallel to the width direction, and a plurality of air intake holes 22 that communicatively connect the inside of the form die 21 and the substantially closed space S are formed in the bottom section 21a.

Further, the substantially closed space S inside the rotating drum 20 is divided into zones by a division wall 27 in the circumferential direction Dc as shown in FIG. 3A, and an exhaust duct 41 is connected to a first zone Z1 shown in FIG. 3A. And air inside the first zone Z1 is exhausted from an exhaust opening section 42 of the exhaust duct 41 and thereby the first zone Z1 is maintained in a negative pressure state lower than a pressure of outside air. Thus, air is sucked through the air intake holes 22 of the form die 21 when the form die 21 moves by a position on the outer circumferential surface 20a that corresponds to the first zone Z1. However, a second zone Z2 is not connected to the exhaust duct 41, therefore the air intake of the form die 21 nearly stops when the form die 21 enters the position on the outer circumferential surface 20a that corresponds to the second zone Z2. The supply opening section 31a of the supply duct 31 is disposed in the first zone Z1, and a release position Pf for releasing the absorbent body 1 from the form die 21 is set in the second zone Z2.

The supply duct 31 is a tubular member with an approximately rectangular cross section and is disposed obliquely above the rotating drum 20. The supply opening section 31a in a lower end of the supply duct 31 covers an approximately obliquely upper portion of the outer circumferential surface 20a of the rotating drum 20 over a predetermined range. Moreover, the pulp fibers 2 that have been pulverized by a pulverizing device (not shown) or the like are supplied from an upper end opening 31b of the supply duct 31 along with air flow 3. Thus, inside the supply duct 31, the air mixture 3 which the pulp fibers 2 are mixed therein flows towards the lower supply opening section 31a. Also in some cases, a polymer casting pipe 38 can be disposed inside the supply duct 31 as shown in FIG. 3A, and can discharge superabsorbent polymer 5 from a casting opening 38a thereof towards the outer circumferential surface 20a.

In a fiber stacking apparatus 10 with such configuration, the absorbent body 1 is formed on a sheet-like member 4 such as nonwoven fabric as follows. First, as shown in FIG. 3A, the continuously supplied sheet-like member 4 is wrapped around the outer circumferential surface 20a of the rotating drum 20 at a position Ps in the downstream side of the supply duct 31 in the circumferential direction Dc. The sheet-like member 4 on the outer circumferential surface 20a is moved toward the downstream side in the circumferential direction Dc by the rotational movement of the rotating drum 20 with the outer circumferential surface 20a without slipping.

On the other hand, in the case where the form die 21 on the outer circumferential surface 20a, by a driving movement of the rotating drum 20, passes the position of the supply duct 31, the air mixture 3 discharged and supplied from the supply opening section 31a is sucked through the air intake holes 22 of the form die 2. But at that time the pulp fibers 2 passing through the air intake holes 22 are restricted by the sheet-like member 4 on the outer circumferential surface 20a, and thereby the pulp fibers 2 in the air mixture 3 stack at the position in the sheet-like member 4 contacting the bottom section 21a of the form die 21 and are formed into the absorbent body 1. When the form die 21 finishes passing the position of the supply opening section 31a and reaches the release position Pf where the outer circumferential surface 20a faces downward, the sheet-like member 4 is released from the outer circumferential surface 20a by roller 24 provided in the release position Pf, and thereby the absorbent body 1 is released from the form die 21 and is placed on the sheet-like member 4. The absorbent body 1 is formed in this way.

By the way, the exhaust duct 41 for keeping the first zone Z1 of the rotating drum 20 in a negative pressure state is a circular pipe with cross-section of a perfect circle having an opened pipe end 41a. A pipe axis direction C41 of the exhaust duct 41 is aligned with the width direction of the rotating drum 20, and the exhaust duct 41 is connected to one of the wall section 25 of the pair of circular wall sections 25, 25.

However, in such case, as shown in FIG. 4, if the exhaust opening section 42 as a pipe end opening of the exhaust duct 41 is provided flush with an inner wall face 25a of the circular wall section 25, sucking-pressure distribution inside the form die 21 gets biased in the width direction. That is, in an example in FIG. 4, sucking pressure on the right side of the form die 21 is higher, while sucking pressure on the left side is lower. As a result, the right side of the absorbent body 1 stacked inside the form die 21 gets higher and the left side gets lower, that is, a stack distribution of the absorbent body 1 gets biased in the width direction.

To prevent this, in the first embodiment, as shown in FIG. 3B, the exhaust opening section 42 at the pipe end 41a of the exhaust duct 41 is projected inward to the closed space S beyond the circular wall section 25. That is, the exhaust opening section 42 is positioned inside the inner wall face 25a of the circular wall section 25 in the width direction. Thus the sucking-pressure distribution inside the form die 21 is prevented from being biased in the width direction.

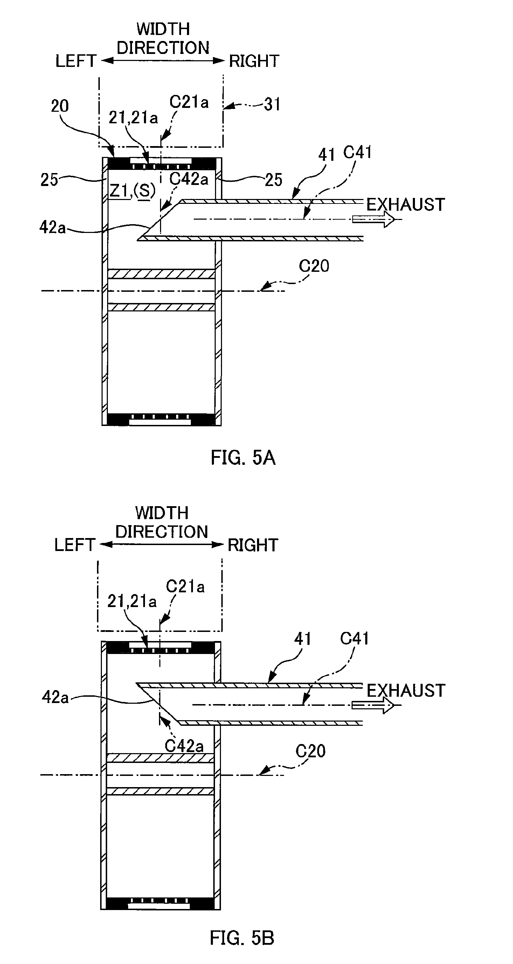

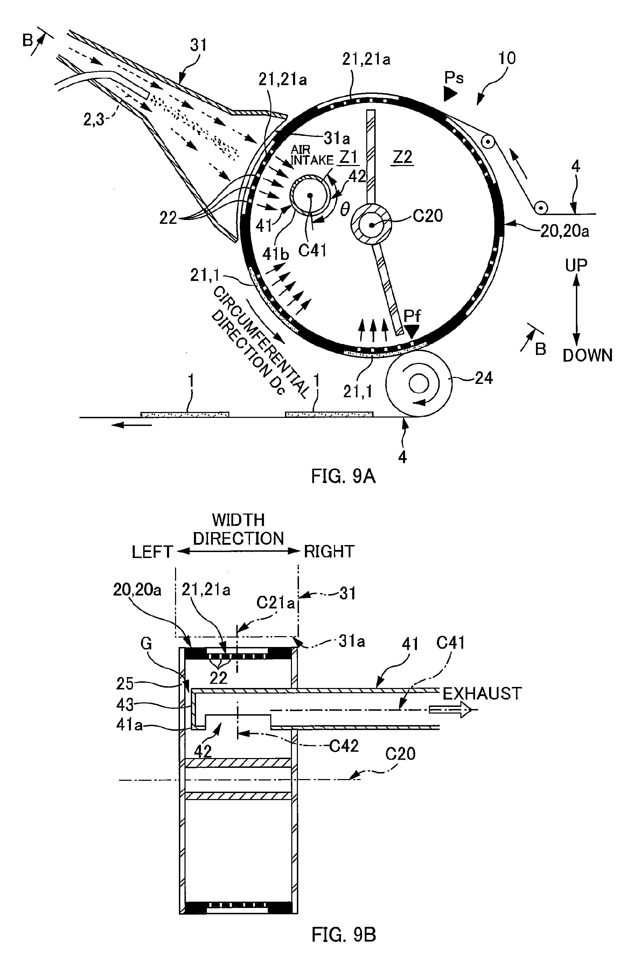

Further, in an example of FIG. 3B, the exhaust opening section 42 is made by forming an opening at the pipe end 41a by cutting the exhaust duct 41 along an orthogonal direction of the pipe axis direction C41. However, there is no limitation to this and an exhaust opening section 42a can be made by forming an opening at the pipe end by cutting the exhaust duct 41 in a slanting direction at an angle besides 90 degrees with respect to the pipe axis direction C41 as shown in FIG. 5A. In such case, rather than facing the exhaust opening section 42a to the supply opening section 31a of the supply duct 31 as shown in FIG. 5A it is preferable to face the exhaust opening section 42a in the opposite direction as shown in FIG. 5B. Here, explanation of reason for this is omitted since it is the same as examples of FIGS. 9A and 9B of second embodiment described later.

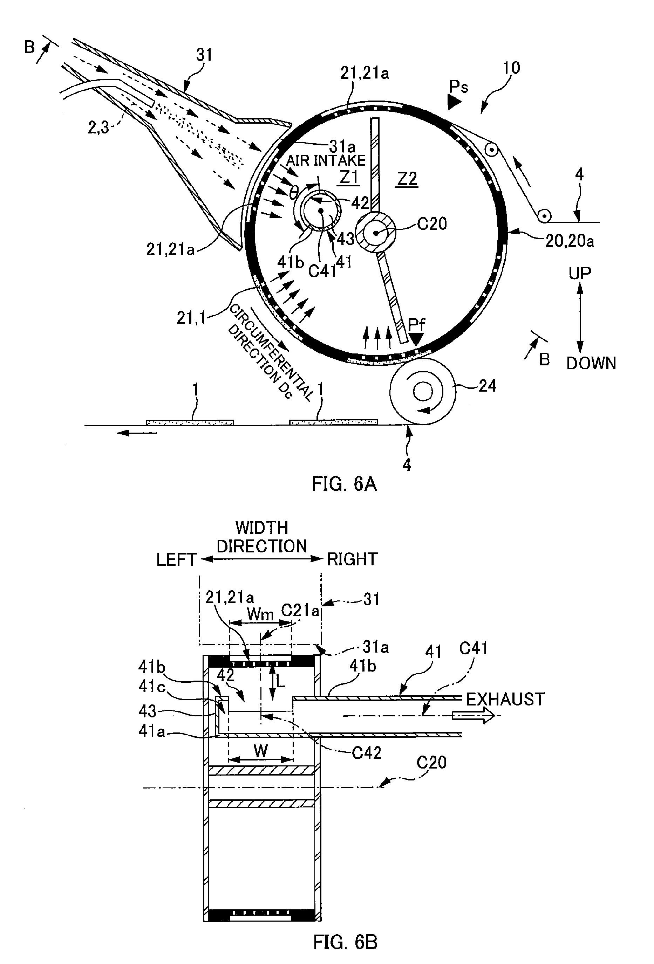

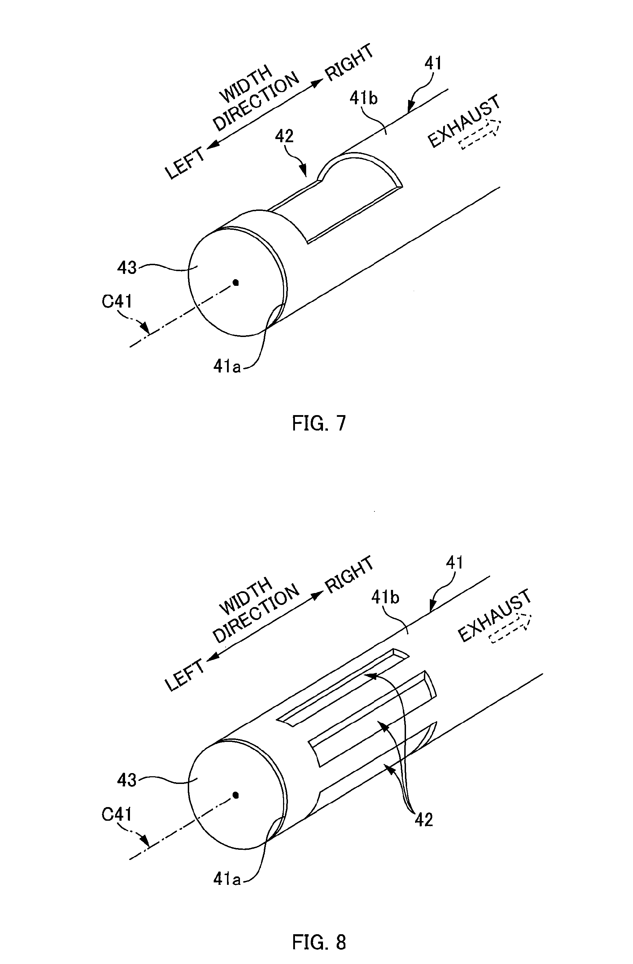

FIGS. 6A, 6B and 7 are explanatory diagrams of the second embodiment of the exhaust duct 41. FIG. 6A is a center vertical cross-sectional view of the exhaust duct 41. FIG. 6B is a cross-sectional view taken from line B-B of FIG. 6A. FIG. 7 is a perspective view of the exhaust duct 41.

The exhaust duct 41 of the second embodiment differs from the first embodiment on the point that the forming position of the exhaust opening section 42. That is, a plate-like blocking lid 43 (corresponds to a blocking member) is abutting against the pipe end 41a of the exhaust duct 41 and the pipe end opening is blocked thus ventilating is not possible. And instead, a rectangular exhaust opening section 42 is formed by cutting out a circumferential wall section (pipe wall section) 41b of the exhaust duct 41 at an angular range θ of 150° in the circumferential direction.

Thus, as shown in FIG. 6B, a distance L between the bottom section 21a of the form die 21 and the exhaust opening section 42 can be made equal over the width direction, and as a result, the stack distribution of the absorbent body 1 can be made uniform in the width direction. And as shown in FIG. 6A, in the case of the form die 21 passing the adjacent position of the exhaust opening section 42, the exhaust opening section 42 can suck air along the direction substantially normal to the bottom section (face) 21a of the form die 21 shown in FIG. 6B. And the stack distribution of the absorbent body 1 can be made uniform also in this way. Further, in the exhaust opening section 42, the air is sucked along the direction substantially normal to the bottom section 21a of the form die 21 as described, therefore several effects from adjusting the air flow can be expected to some extent at the position of the exhaust opening section 42. However the exhaust opening section 42 is disposed by being separated from the bottom section 21a of the form die 21 by a distance L. Thus the effect of adjusting air flow by the exhaust opening section 42 is transmitted gently and acts on the bottom section 21a of the form die 21, and stability of sucking-pressure distribution becomes superior by suppressing a rapid change in the air flow.

Also, it is preferable that the width W of the exhaust opening section 42 is wider than the width Wm of the bottom section 21a of the form die 21. In such respect, sucking-pressure at the bottom section 21a is further uniformed.

Note that, in the examples of FIGS. 6A and 7, the exhaust opening section 42 is formed in one position of the exhaust duct 41 in the circumferential direction. In detail, in the circumferential wall section 41b of the exhaust duct 41, only one exhaust opening section 42 is formed at a position facing the supply opening section 31a of the supply duct 31. However, the position and number of the exhaust opening section 42 formed on the circumferential wall section 41b is not limited to this, and for example, a plurality of exhaust opening sections 42 can be formed by being lined up in the circumferential direction of the exhaust duct 41 as shown in FIG. 8.

However, in the case where only one exhaust opening section 42 is formed, it is preferable that the exhaust opening section 42 is formed on a portion on an opposite side of the portion facing the supply opening section 31a of the supply duct 31 as shown in FIGS. 9A and 9B. In this way, an air flow that has passed the supply opening section 31a and the air intake holes 22 in the bottom section 21a of the form die 21 is sucked through the exhaust opening section 42 after encircling the exhaust duct 41 to the opposite side of that position. Thus, even in the case where the sucking pressure in the exhaust opening section 42 varies to some degree in the width direction, such pressure variation is alleviated at the position of the form die 21 and becomes smaller, that is, the sucking-pressure distribution in the form die 21 in the width direction is made uniform.

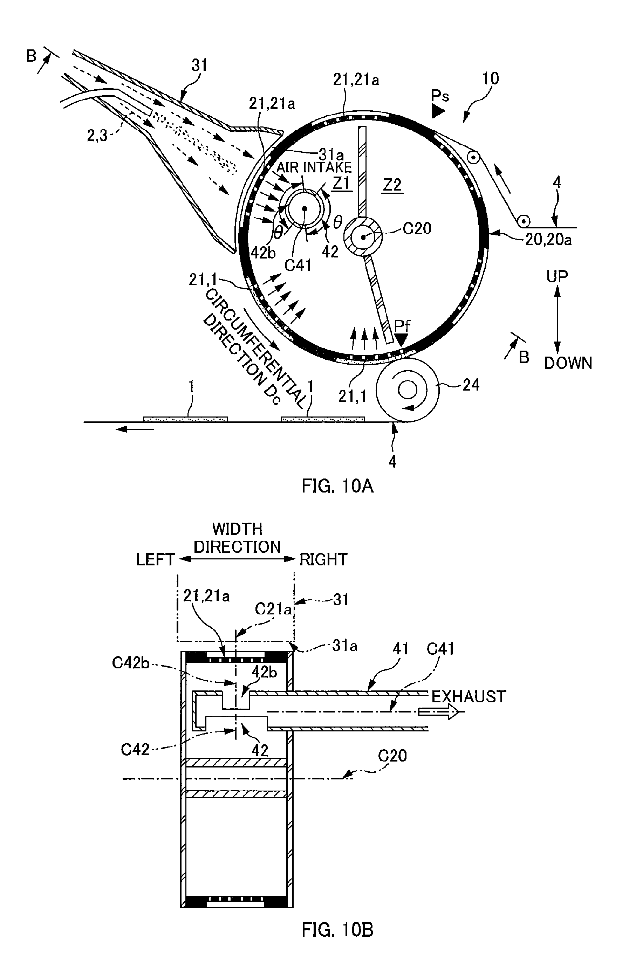

By the way, in the case where forming position of the exhaust opening section 42 in the circumferential wall section 41b is far from the supply opening section 31a as shown in FIGS. 9A and 9B, loss of pressure becomes large compared to the example of FIGS. 6A and 6B and there is fear that there may be a case that a necessary pressure level cannot be kept at the bottom section 21a of the form die 21. However, in such case, it is preferable that an exhaust opening section 42b with a smaller opening area than the exhaust opening section 42 (hereafter referred to as an auxiliary exhaust opening section 42b) is formed accessorily at the portion facing the supply opening section 31a of the supply duct 31, as shown in FIGS. 10A and 10B. In such case, it is preferable to set the opening-area ratio of the auxiliary exhaust opening section 42b (corresponds to second exhaust opening section) to the exhaust opening section 42 (corresponds to first exhaust opening section) within a range from 10% to 30%. In this way, while efficiently maintaining the effect of uniformizing the sucking-pressure distribution in the form die 21 in the width direction caused by air sucking from an exhaust opening section 42 farther away, decrease of air sucking amount caused by the loss of pressure that may occur is efficiently covered. Also, it is preferable to align a center position C42b of the auxiliary exhaust opening section 42b in the width direction with a center position C42 of the exhaust opening section 42 (FIG. 10B). And thereby, uniform sucking-pressure distribution formed by the exhaust opening section 42 is not disturbed by the auxiliary exhaust opening section 42b.

Further, in the case where the exhaust opening section 42 faces an opposite side of the supply opening section 31a as shown in FIG. 9B, it is preferable to provide a space G between the blocking lid 43 at the pipe end 41a of the exhaust duct 41 and the circular wall section 25 that faces the blocking lid 43. In this way, the air flow from the bottom section 21a of the form die 21 smoothly encircles to flow into the exhaust opening section 42 farther away and as a result, loss of pressure at the exhaust opening section 42 can be further reduced.

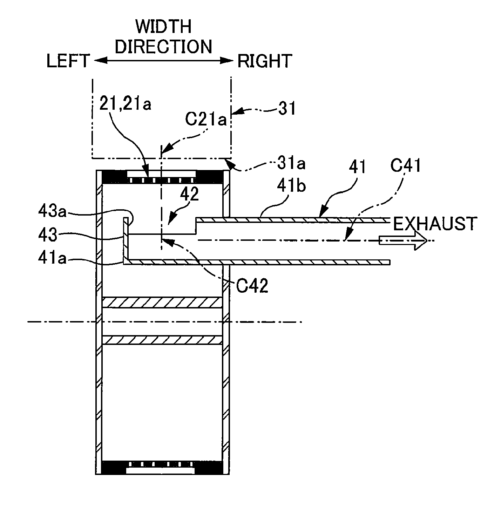

Further, as shown in FIG. 6B, it is preferable that the circumferential wall section 41b exists between the pipe end 41a of the exhaust duct 41 and the exhaust opening section 42 so as to form an air stagnation at a pipe end section 41c of the exhaust duct 41. In this way, decrease of the flow rate of the air flow sucked into the exhaust opening section 42 at the pipe end 41a side is reduced by the air stagnation at the pipe end section 41c, and as a result, sucking-pressure distribution in the exhaust opening section 42 in the width direction can be made uniform. Here, frictional resistance and the like is considered as a reason of decrease in the flow rate of air flow at the pipe end 41a side. And this frictional resistance is applied by a board face 43a to the air flow, based on viscosity of the air, when the air flow flows near the board face 43a of the blocking lid 43 that blocks the pipe end opening of the exhaust duct 41 as shown in FIG. 11. Thus, by forming the air stagnation at the pipe end section 41c as shown in FIG. 6B, the air stagnation functions as a buffer space that separates the board face 43a and the air flow. And as a result, decrease of the flow rate of the air flow at the pipe end 41a side is reduced efficiently.

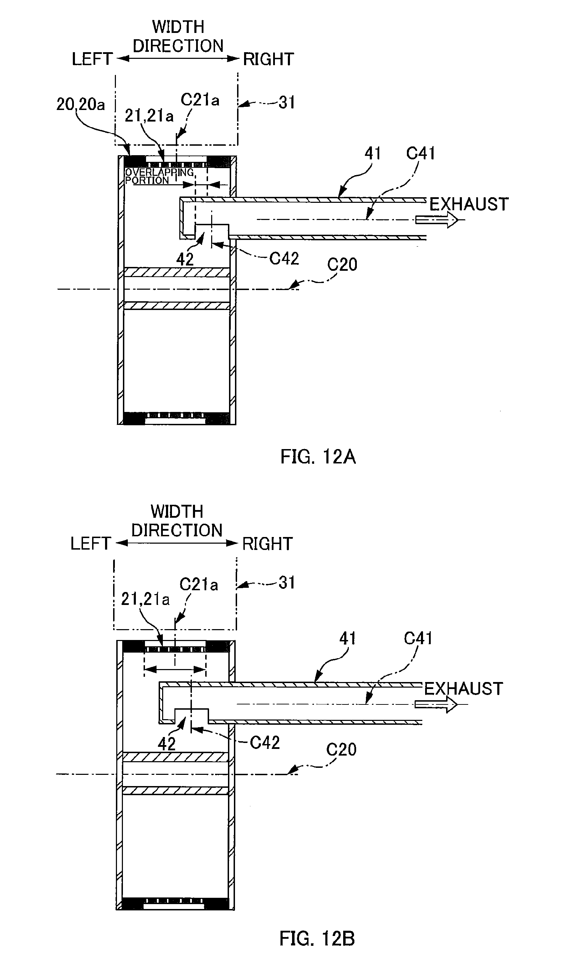

By the way, in the examples according to the first embodiment (FIGS. 3B, 5A, and 5B) and the examples according to the second embodiment (FIGS. 6B, 9B, and 10B), the cases in which the center position C42 (or C42a, C42b) of the exhaust opening section 42 in the width direction aligns with the center position C21a of the bottom section 21a of the form die 21 were shown as examples. However, these are the most preferable examples, that is, the exhaust opening section 42 can be shifted in the width direction from such positional relationship. However, it is not preferable if the position is largely shifted and as an allowable range, as shown in FIG. 12A, at least the bottom section 21a of the form die 21 and the exhaust opening section 42 should be overlapped in the width direction. And as an allowable range that is further preferable, as shown in FIG. 12B, the center position C42 (or C42a, C42b) of the exhaust opening section 42 in the width direction should be positioned in the bottom section 21a of the form die 21. And when positioned in such a way, in the case of the form die 21 passing a position adjacent to the exhaust opening section 42, air is sucked reliably along the direction substantially normal to the bottom section 21a of the form die 21 and thus the stack distribution of the absorbent body 1 is made uniform.

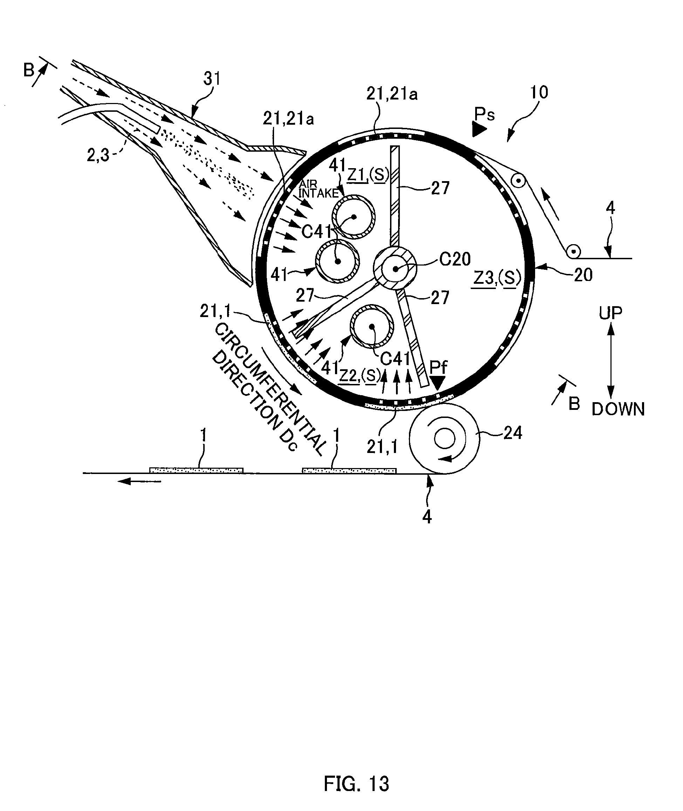

The number of the exhaust duct 41 is not limited to one as described above, and a plurality of the ducts can be provided. For example, as shown in FIG. 13, in the case where the substantially closed space S on the inner circumferential side of the rotating drum 20 is divided into three zones, that are from the first zone to the third zone (Z1, Z2 and Z3), by the division wall 27, the exhaust duct 41 can be provided individually to each of the first zone Z1 facing the supply opening section 31a of the supply duct 31, and the second zone Z2 at the downstream in the circumferential direction Dc.

### Other embodiments

In the description above, embodiments of the invention were described. However, the invention is not limited to these embodiments, and modifications as described below are possible.

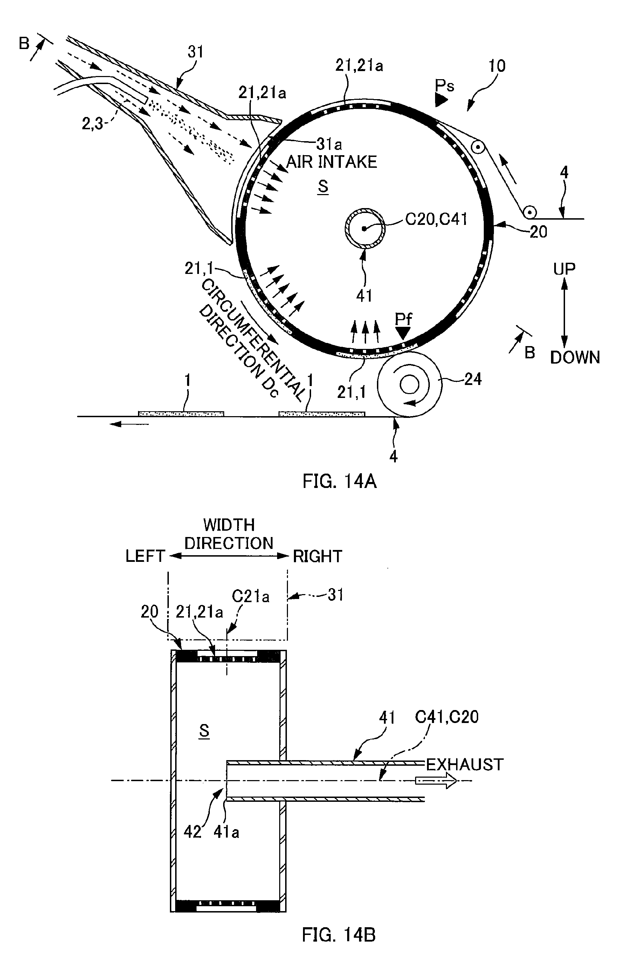

In the foregoing embodiment, the rotating drum 20 to which the exhaust duct 41 can be selectively connected in units of zones Z1 and Z2 that are partitioned at the inner circumferential side of the rotating drum 20, that is, the rotating drum 20 to which the exhaust duct 41 is connected at the position decentered from the rotation center axis C20 of the rotating drum 20 is shown as an example. However, there is no limitation to this. For example, as shown in FIGS. 14A and 14B, the invention can be applied to the rotating drum 20 of which the exhaust duct 41 is disposed concentrically with the rotation center axis C20 of the rotating drum 20.

In the foregoing embodiment, the circular pipe with a perfect circle cross-section is shown as an example of a member of the exhaust duct 41. However, there is no limitation to this and any shape is possible as long as it is a tubular member. For example, a round pipe with an oval cross-section, and a pipe with a polygonal cross-section such as a square pipe may be used.

In the foregoing second embodiment, the rectangular exhaust opening section 42 was formed in the circumferential wall section 41b of the exhaust duct 41. However, there is no limitation to this and for example, an opening with circular-shape such as a perfect circle or an oval, or an opening with a polygonal-shape such as triangle are possible. Also, the exhaust opening section 42 can be configured as a group of throughholes consisting of a plurality of small throughholes densely disposed.

In the foregoing second embodiment, the rectangular exhaust opening section 42 and the auxiliary exhaust opening section 42b were formed by cutting out the circumferential wall section 41b of the exhaust duct 41 in the angular range θ of 150° in the circumferential direction. However the angular range θ is not limited to this and it can be changed appropriately.

In the foregoing embodiment, nothing was interposed between the bottom section 21a of the form die 21 of the rotating drum 20 and the exhaust opening section 42. However, it is possible to provide an air flow adjuster such as a mesh body between the bottom section 21a and the exhaust opening section 42 so as to adjust the air flow to further uniform the air-sucking-pressure distribution in the form die 21.

In the foregoing embodiment, the pipe axis direction C41 of the exhaust duct 41 was disposed along the width direction of the rotating drum 20. However the pipe axis direction C41 can be tilted to some degree from the width direction as long as the exhaust duct 41 is connected to the circular wall section 25 that cover openings at both ends of the rotating drum 20.

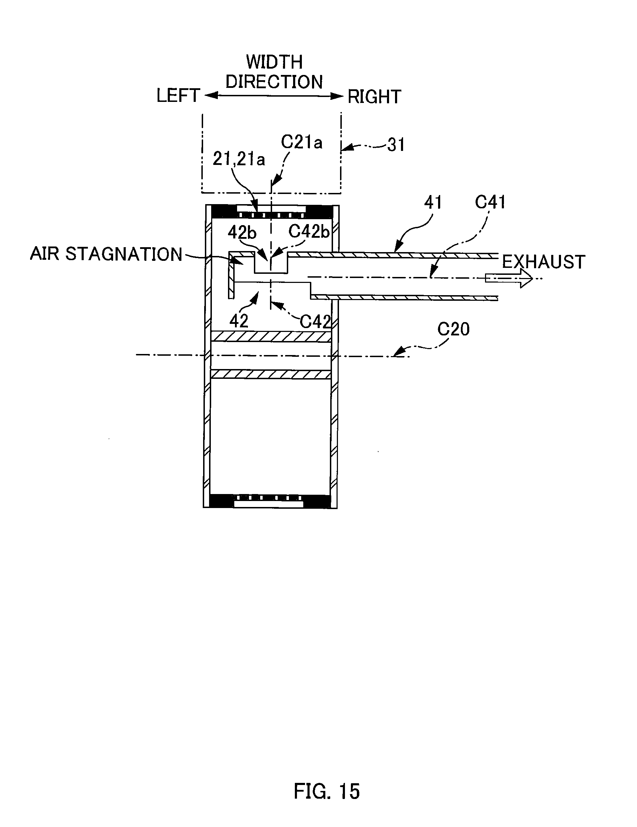

In an example of FIG. 10B of the foregoing second embodiment, the configuration in which the air stagnation is formed at the pipe end section 41c of both of the exhaust opening section 42 and the auxiliary exhaust opening section 42b was shown as an example. However, there is no limitation to this and as shown in FIG. 15, it is possible that the air stagnation is formed only at the auxiliary exhaust opening section 42b and not formed at the exhaust opening section 42.

In the foregoing embodiment, the air 3 was given as an example of a gas discharged and supplied from the supply duct 31. However, there is no limitation to this and the air 3 may be any sort of gas as long as it can be mixed with the fluid absorbent fiber without causing any chemical reaction with the fiber, thus nitrogen or the like is possible.

In the foregoing embodiment, the configuration in which the form die 21 is formed on the outer circumferential surface 20a of the rotating drum 20, and the moving path of the form die 21 is the circumferential direction Dc of the rotating drum 20 was shown as an example. However, there is no limitation to this and any configuration is possible as long as the form die 21 moves in one direction along a predetermined moving path.

For example, a belt of a conveyer belt can be used as a forming member. Specifically, first, the recessed form dies 21 are formed on the belt face (corresponds to a predetermined face) of the belt, and the belt is moved in a predetermined orbit. The supply duct 31 is disposed in a predetermined position on the orbit, and the negative pressure room partition member for partitioning the negative pressure room cooperatively with the belt face is provided in the position on an opposite side of the supply opening section 31a of the supply duct 31 across the belt face. The negative pressure room partition member is, for example, a substantially rectangular box with a wall section facing the belt face detached. And the exhaust duct is connected to, of the wall sections configuring the box, one wall section of a pair of wall sections disposed by sandwiching the orbit between them in the width direction of the belt face.

In the foregoing embodiment, the pulp fibers 2 (pulp that has been pulverized into fibers) were described as an example of the fluid absorbent fibers. However, cellulose such as cotton, regenerated cellulose such as rayon and fibrillated rayon, semisynthetic cellulose such as acetate and triacetate, fibrous polymers, and thermoplastic fibers may also be used, or may also be used in combination.

### Reference Signs List

1: absorbent body, 2: pulp fibers(fluid absorbent fibers), 3: air mixture (gas, air flow), 4: sheet-like member, 5: superabsorbent polymer, 10: fiber stacking apparatus (apparatus for manufacturing absorbent body), 10a: fiber stacking apparatus, 10b: fiber stacking apparatus, 20: rotating drum (forming member), 20a: outer circumferential surface (predetermined face), 21: form die, 21a: bottom section, 22: air intake holes, 24: roller, 25: circular wall section (wall section, negative pressure room partition member), 25a: inner wall face, 26: cylindrical division wall, 27: division wall, 31: supply duct, 31a: supply opening section, 31b: upper end opening, 38: polymer casting pipe, 38a: casting opening, 41: exhaust duct, 41a: pipe end, 41b: circumferential wall section, 41c: pipe end section, 42: exhaust opening section (first exhaust opening section), 42a: exhaust opening section (first exhaust opening section), 42b: auxiliary exhaust opening section (second exhaust opening section), 42d: exhaust opening section, 43: blocking lid (blocking member), 43a: board face, Dc: circumferential direction, Pf: release position, Z1: first zone, Z2: second zone, G: gap, S: substantially closed space (negative pressure room)

## Claims

1. An apparatus for manufacturing an absorbent body, comprising:
a forming member having a predetermined face with a recessed form die formed thereon, moving the form die in one direction along a moving path intersecting a width direction of the predetermined face;
a supply duct disposed in a predetermined position in the moving path, supplying a gas including fluid absorbent fibers from a supply opening section toward the predetermined face;
a negative pressure room partition member provided on a side of the predetermined face, with the supply opening section provided on an opposite side of the predetermined face, the negative pressure room partition member partitioning a negative pressure room in cooperation with the predetermined face; and
an exhaust duct exhausting a gas inside the negative pressure room so as to create a negative pressure inside the negative pressure room,
wherein in a case of the form die passing a position of the supply opening section, by a gas from the supply duct being sucked through air intake holes in a bottom section of the form die into the negative pressure room, the fluid absorbent fibers in the gas are stacked in the form die to form an absorbent body,
the apparatus further having
the negative pressure room partition member including a pair of wall sections disposed by sandwiching therebetween the moving path in the width direction, the negative pressure room being partitioned between the pair of wall sections, and
the exhaust duct connected to one wall section of the pair of wall sections, with an exhaust opening section of the exhaust duct provided by projecting inward beyond the wall section into the negative pressure room.

2. An apparatus for manufacturing an absorbent body according to claim 1,
wherein the exhaust duct is a tubular member and
the exhaust opening section is formed in a circumferential wall section of the exhaust duct.

3. An apparatus for manufacturing an absorbent body according to claim 2,
wherein a pipe axis direction of a portion of the exhaust duct positioned in the negative pressure room is parallel to the predetermined face and the bottom section of the form die.

4. An apparatus for manufacturing an absorbent body according to claim 2 or 3,
wherein a portion at which the exhaust opening section is formed in a circumferential direction of the exhaust duct is a portion on an opposite side of a portion opposing the supply opening section.

5. An apparatus for manufacturing an absorbent body according to claim 4,
wherein in a case where the exhaust opening section is a first exhaust opening section, a second exhaust opening section is formed in a portion opposing the supply opening section in the circumferential wall section of the exhaust duct.

6. An apparatus for manufacturing an absorbent body according to claim 5,
wherein an opening area of the second exhaust opening section is smaller than an opening area of the first exhaust opening section.

7. An apparatus for manufacturing an absorbent body according to any one of claims 2 to 6,
wherein an end of the exhaust duct in the pipe axis direction is blocked by a blocking member, and
an existence of the circumferential wall section of the exhaust duct between the end and the exhaust opening section causes a gas stagnation at an end portion of the exhaust duct in the pipe axis direction.

8. An apparatus for manufacturing an absorbent body according to any one of claims 1 to 7,
wherein the bottom section of the form die and the exhaust opening section are disposed to at least overlap in the width direction.

9. An apparatus for manufacturing an absorbent body according to any one of claims 1 to 8,
wherein a center position of the exhaust opening section in the width direction is positioned in the bottom section of the form die.

10. An apparatus for manufacturing an absorbent body according to any one of claims 1 to 9,
wherein the center position of the exhaust opening section in the width direction aligns with a center position of the bottom section of the form die.

11. An apparatus for manufacturing an absorbent body according to any one of claims 1 to 10,
wherein the forming member is a cylindrical member that continuously rotates in one circumferential direction,
the recessed form die is formed on an outer circumferential surface of the cylindrical member as the predetermined face, moved by rotation of the cylindrical member in the circumferential direction in a path along the circumferential direction as the moving path,
a pair of circular wall sections are included as the pair of wall sections of the negative pressure room partition member, the pair of circular wall sections covering openings at both ends of the cylindrical member in the width direction to partition the negative pressure room on an inner circumferential side of the cylindrical member, and
the exhaust duct is connected to one wall section of the pair of circular wall sections.

12. A method for manufacturing an absorbent body, comprising:
(A) preparing an apparatus for manufacturing an absorbent body, the apparatus for manufacturing the absorbent body using
a forming member having a predetermined face with a recessed form die formed thereon, moving the form die in one direction along a moving path intersecting a width direction of the predetermined face;
a supply duct disposed in a predetermined position in the moving path, supplying a gas including fluid absorbent fibers from a supply opening section toward the predetermined face;
a negative pressure room partition member provided on a side of the predetermined face, with the supply opening section provided on an opposite side of the predetermined face, the negative pressure room partition member partitioning a negative pressure room in cooperation with the predetermined face; and
an exhaust duct exhausting a gas inside the negative pressure room so as to create a negative pressure inside the negative pressure room,
wherein in a case of the form die passing a position of the supply opening section, by a gas from the supply duct being sucked through air intake holes in a bottom section of the form die into the negative pressure room, the fluid absorbent fibers in the gas are stacked in the form die to form the absorbent body,
the apparatus further having
the negative pressure room partition member including a pair of wall sections disposed by sandwiching therebetween the moving path in the width direction, the negative pressure room being partitioned between the pair of wall sections, and
the exhaust duct connected to one wall section of the pair of wall sections, with an exhaust opening section of the exhaust duct provided by projecting inward beyond the wall section into the negative pressure room; and
(B) manufacturing the absorbent body using the apparatus for manufacturing the absorbent body.
